# EUROPEAN PATENT SPECIFICATION

(11) **EP 2 306 257 B1**
(45) Date of publication and mention of the grant of the patent: **21.11.2012**
(21) Application number: 10009235.2
(22) Date of filing: 06.09.2010
(51) Int. Cl.: A61M 39/22, G05D 16/06, F16K 1/52, G05D 7/01

(54) **Gas pressure reducing and stabilizing device for gas of various kinds for medical uses**
Gasdruckminder- u. Stabilisierungseinrichtung für medizinisch verwendete Gase
Appareil de réduction et de stabilisation de la pression de gaz pour gaz diverses d'applications médicales

(30) Priority: 10.09.2009 IT PN20090049
(43) Date of publication of application: 06.04.2011
(73) Proprietor: MD S.r.l., 30037 Scorzè (VE) (IT)
(72) Inventor: Endrizzi, Dario, 30030 Gardigiano (Ve) (IT)
(74) Representative: Dalla Rosa, Adriano

(56) References cited:
- EP-A1- 1 190 730
- EP-A1- 2 083 255
- WO-A1-2006/011023
- CN-A- 101 476 670

## Description

The invention relates to a gas-pressure reducing and stabilizing device provided with physical stop means intended for use in connection with equipment designed for delivering gases of various kind for medical applications, such device being adapted to enable the pressure of such gases to be reduced in a continuous manner within a pre-established adjustment range and, at the same time, to maintain or cut off in the same device the continuity of the gas flow circulating through the gas inflow and outflow conduits.

From WO 2006/011023 A1 is known a multifunctional valve unit for use on a gas cylinder to which it is attached for the controlled filling of the cylinder and for the controlled discharge of gas therefrom. The unit has a unitary or monolithic valve body providing a filling port and a cylinder connector in gas flow communication with the filling port, for releasable attachment of the unit to a gas cylinder. The unit further comprises a manually operable manual shut-off valve, a gas pressure regulator, a minimum retention valve, a pressure gauge, a flow control device, and a pressure relief safety valve, all of which are mounted on the valve body.

The flow control device is, as a whole, replaceably removable from the remainder of the multifunctional valve unit, while the cylinder connector is connected to a gas cylinder containing gas and the manual shut-off valve is closed.

From EP 2083255 A1 is known a device for drawing gas with a pressure regulator, and a metering valve, in which the pressure regulator and the metering valve are integrated on a common valve body.

From CN 101476670 A is known a gas pressure regulator, provided with a single valve body including a gas inflow conduit and a gas outflow conduit, connected to each other through a gas pressure regulator, and provided with valve means for allowing or stopping the gas flowing from the inflow to the outflow conduit.

From EP 1190730 A1 is known an apparatus for supplying a respiratory gas to a respiratory air way of a patient. The apparatus includes a cylinder filled with a respiratory gas, a cart having an accommodating portion for disposing the cylinder, a shut-off valve attached to the cylinder, a flow regulating valve attached to the shut-off valve, a conduit attached to the flow regulating valve, for directing the respiratory gas to the inlet of the respiratory airway of the patient, a coupler integrally connected to the flow regulator, for coupling the flow regulating valve to the shut-off valve, and a linkage for disengaging the coupler from the shut-off valve when the cylinder is detached from the cart.

As is largely known in the art, health-care structures of surgery and medical treatment facilities, hospitals, clinics and the like are equipped with fixed installations and plants for the delivery and the circulation of medical gases of various kind that are used to treat patients in need, such gases including for instance oxygen, medical air, nitrous oxide, carbon dioxide, oxygen/nitrous oxide mixtures, nitrogen and air for instrumental uses, anaesthetic gases, and the like. Such installations are generally designed to branch off respective supply sources for the supply of the medical gases needed for the application, wherein such gas supply sources are usually located on the outside of the health-care structures themselves, and to reach into the related buildings through conduits formed of copper piping. The resulting gas distribution networks are provided with related shut-off means both at the central supply and control station and with the aid of shut-off valves located inside cabinets provided at the base of the risers, i.e. vertical pipes, and at the site of any possible branch point. Depending on the kind of installation used, the gas distribution pressure can be reduced and stabilized directly at the central supply and control station to the value at which the medium has to be delivered to the patient being treated, or can be handled in each single compartment or zone of the health-care structure by means of a second-stage pressure reducer provided to serve such single, specific hospital area.

The installation extends up to and ends at the terminal distribution units, which are either wall-mounted or are mounted on appropriate technical units, such as suspended cabinets or bed-head beams. The medical or nursing staff is able to connect to such terminal units, via appropriate plug-in attachments, to derive the medical gas from the installation and deliver it to the patient. Area or zone valves provided with physical shut-off or stop means are located upstream to said terminal units to provide control of the various hospital or health-care areas.

Gas distribution pressures are monitored in a continuous manner through proper alarm signals, in the same way as are monitored through related alarm signals the gas supplies available at the central supply and control station.

Each gas-pressure reducing device is substantially formed of an aluminium body in a single-piece construction, as obtained by machining on chip-making machine tools followed by an anodizing finishing treatment. Inside such body there is accommodated the actual pressure regulation member, which is comprised of an impermeable membrane and a counter spring, the setting of which can be varied in a continuous manner within pre-established limits, so as to each time adjust, i.e. reduce the pressure of the gas being delivered to the desired or required values. Such members can be isolated by means of shut-off valves located upstream to and downstream from them, as referred to the flow direction of the gases, so as to allow for quick and convenient repair or replacement of those parts that are subject to wear and tear, wherein such valves can be operated manually and alternately from an opened position to a closed position so as to allow the gas outflowing from the pressure regulator at a reduced pressure to flow further on towards the conduits branching off therefrom, and connected to one or more outlets of the pressure regulator on one side and the apparatuses used to deliver the medical gases to the patients to therapeutic purposes, or to relieve pains caused by illness as the case may be, on the other side and prevent such gas from flowing further on, respectively.

The present invention relates to a gas-pressure reducing and stabilizing device realised in a manner different than that of the references and provided with physical stop or shut-off facility for gas distribution installations intended for the delivery of medical gases of various kinds, which is not only adapted to reduce, i.e. regulate within pre-set limits the pressure of the medical gases due to be delivered to the patients, but also to concurrently work so as to maintain or cut off in the same device the continuity of the gas flow circulating through the gas supply inflow conduits and the outflow conduits leading to the apparatuses provided to deliver the same gases to the patients.

The inventive gas-pressure reducing and stabilizing device provided with physical stop or shut-off facility incorporates the characteristics and features as substantially described below, with particular reference to the appended patent claims.

Features and advantages of the present invention will anyway be more readily understood from the following description, given to sole illustrative purposes by way of non-limiting example with reference to the accompanying drawings, wherein:
- Fig. 1 is a front view of the gas-pressure reducing and stabilizing device according to the present invention, as shown in a first embodiment thereof;
- Fig. 2 is a perspective front and top view of the gas-pressure reducing and stabilizing device shown in Figure 1;
- Fig. 3 is a perspective front and bottom view of the gas-pressure reducing and stabilizing device shown in Figure 1;
- Fig. 4 is a perspective front and top view of the gas-pressure reducing and stabilizing device according to the present invention, as shown with a pressure-regulating part in a disassembled state thereof and in a second embodiment of the same device;
- Fig. 5 is a side view of the device shown in Figure 4;
- Fig. 6 is a front view of the device shown in Figure 5, as sectioned along the line C-C;
- Fig. 7 is a perspective exploded front view of the pressure-regulating part of the gas-pressure reducing and stabilizing device according to the present invention, such part being also adapted to physically shut-off the installation for the distribution of medical gases;
- Fig. 8 is a front cross-sectional view of the gas-pressure component part shown in Figure 7, as set in an opened operating position thereof for enabling the medical gas to pass therethrough at a reduced pressure and flow towards the apparatuses for the delivery of the gas to the patients;
- Fig. 9 is a front cross-sectional view of the gas-pressure component part shown in Figure 7, as set in the closed operating position thereof for preventing the medical gas at a reduced pressure from passing therethrough and flowing on towards the apparatuses for the delivery of the gas to the patients;
- Fig. 10 is a perspective front view of the gas-pressure reducing and stabilizing device according to the present invention, as shown in two different operating steps a), b) and c), d) thereof, which are required to have the gas-pressure regulating part inserted into and extracted from the body of the device, respectively.

Illustrated in the above-listed Figures there is the gas-pressure reducing and stabilizing device for the delivery of medical gases according to the present invention, which is adapted to be removably mounted in gas distribution and delivery equipment and installations, which are designed for delivering gases of various kind for medical applications as installed in health-care structures of surgery and medical treatment facilities, hospitals, clinics and the like, to the purpose of enabling the pressure of the medical gases used in such installations to be reduced and stabilized in a continuous manner within a pre-established adjustment range, so as to be able to deliver such gases at such reduced pressure values to the patients being treated, the device being adapted to at the same time maintain or cut off in the same device the continuity of the gas flow circulating through the gas inflow and outflow conduits.

As usual in the art, the medical gases used and circulating in the gas distribution and delivery installations of the above-noted kind may for instance include oxygen, medical air, nitrous oxide, carbon dioxide, oxygen/nitrous oxide mixtures, nitrogen and air for instrumental uses, anaesthetic gases, and the like.

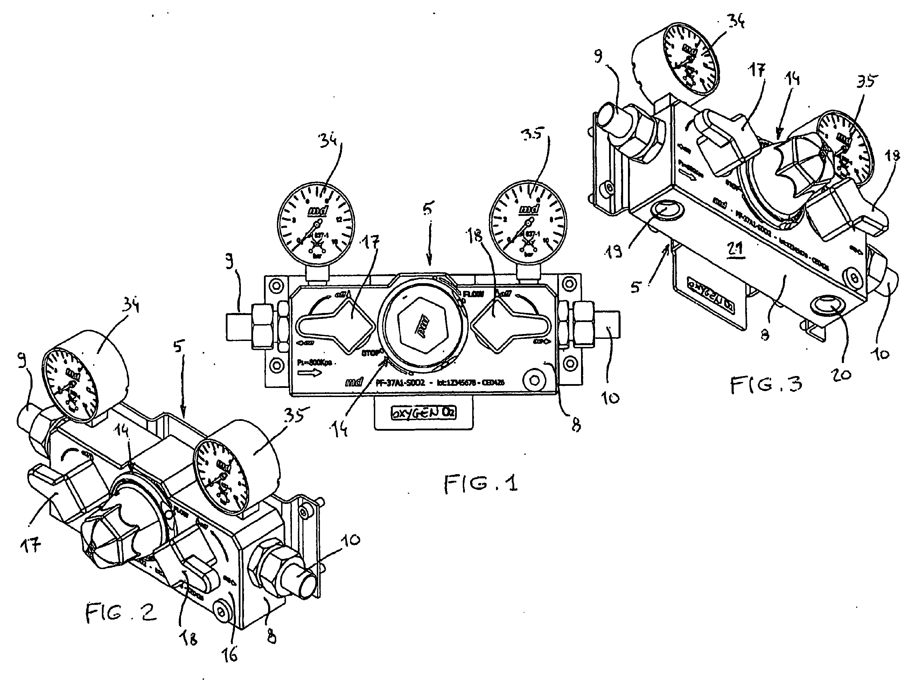

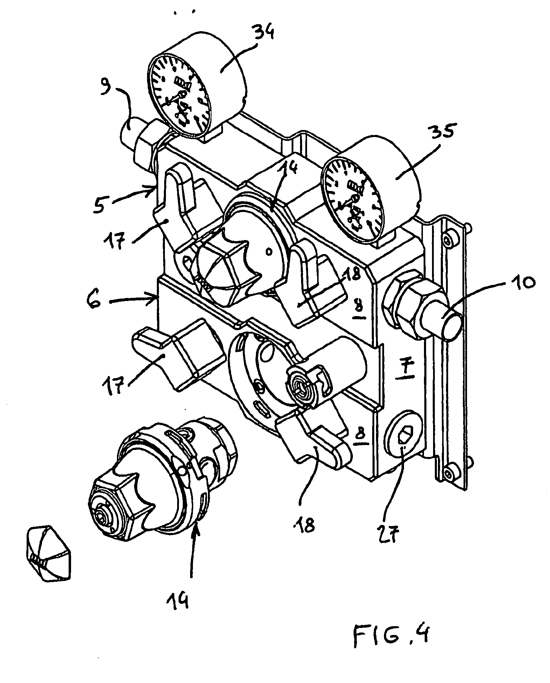

In particular, Figures 1 through to 3 can be noticed to illustrate the gas-pressure reducing and stabilizing device provided with physical shut-off or cut-out means according to the present invention, as shown in a first embodiment thereof, wherein such device can be noticed to be comprised of a single device 5 adapted to supply medical gas at a reduced pressure to one or more gas delivery apparatuses (not shown), whereas illustrated in Figure 4 there is the same inventive device in a second embodiment thereof, wherein such device is in this case formed of two separate devices 5 and 6, which are identical to each other and are mounted on a common support structure 7 in a position in which they lie above each other, the inner conduits of such devices are fluidly communicating with each other, in the way that shall be described in greater detail further on, so as to equally supply medical gas at a reduced pressure to one or more gas delivery apparatuses (not shown, either). Referring to these Figures, there can be noticed that each one of the above-mentioned gas-pressure reducing and stabilizing devices 5 and 6 is substantially comprised of a respective case-like body 8 made of metal material in a single-piece construction, wherein such metal material is preferably aluminium and the case-like body itself is manufactured by machining on chip-making machine tools and subsequently finished by an anodizing process. Such body has a length that is greater than the height and the thickness thereof and on a side thereof (the left side in the example being considered) there is provided an inflow port 9 adapted to be connected in a traditional manner to the gas supply conduit (not shown) that is in turn connected to the supply source of the related medical gases being used, which are generally supplied at a quite high supply pressure, so that such pressure has necessarily to be reduced in the above-mentioned gas-pressure reducing and stabilizing device prior to the gas being able to be delivered to the patents under treatment. In turn, on the other side of the afore-cited case-like body 8 lying opposite to the inflow side considered above, there is provided an outflow port 10 that is adapted to be connected in a traditional manner - via a conduit for the distribution of gas at a reduced pressure (not shown) - to at least one traditional apparatus (not shown, either) for the delivery of the medical gas to the patients under treatment.

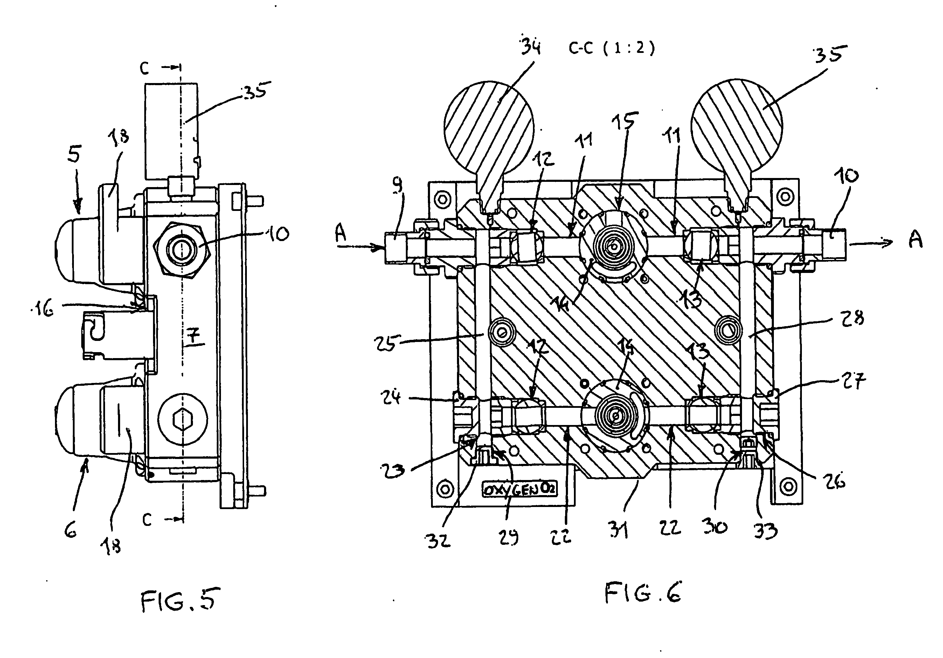

The inflow port 9 and the outflow port 10 are in fluid communication with each other via an inner conduit 11 of the case-like body 8 that is horizontally aligned with both such ports (see Figure 6) and is provided with shut-off means consisting of both a first and a second valve member 12 and 13, located downstream from the inflow port 9 and upstream to the outflow port 10, respectively, relative to the flow direction A of the medical gas circulating therethrough, and a regulating component part 14 that is made and operates in the way that shall be described in greater detail further on, wherein such regulating part is housed in a respective circularly-shaped accommodation 15 provided inside the case-like body 8 in a central position along said inner conduit 11, and capable of being accessed to the front surface 16 of the body (see Figure 5), said accommodation extending down along part of the wall thickness of the same body. Each one of the above-mentioned valve members 12 and 13 is advantageously provided in the form of a revolving ball valve hinged on in the related portion of inner conduit in the case-like body 8 and capable of being actuated by a corresponding rotating lever 17 and 18 hinged externally on the front surface 16 of the same case-like body and displaceable through a 90-deg. rotation from a closed position, as shown in Figure 6, in which the flow of the gas through the inner conduit 11 towards the outflow port 10 is shut-off, to an opened position (not shown), in which the gas is on the contrary allowed to freely flow through the inner conduit 11, the gas-pressure regulating component part 14 of device and, finally, the outflow port 10. In the case of the embodiment contemplating the use of just a single device 5, such as shown in the Figures 1 to 3, the inflow port 9 and the outflow port 10 are further fluidly communicating with a related vertical inner conduit (not shown) terminating in corresponding lower access bores 19 and 20 extending through the bottom surface 21 of the case-like body 8, which may or may not be connected with at least one external emergency medical-gas supply source (not shown), to be used in the case of a temporary failure of the main medical-gas supply, as due to any of a variety of possible causes, or in the case of possible problems due to gas leakages or losses in the main gas supply line, wherein said bores may also be connected to at least a highest and lowest allowable pressure measuring and detecting device (not shown), such as a correspondingly set and rated pressure switch, to have any possible alarm condition in the supply pressure of the gas duly indicated.

In the case that the above-mentioned accessory devices should not be connected to said vertical inner conduits, the related access bores are plugged in a gas-tight manner by means of appropriate plug-in caps or the like (not shown), thereby positively preventing medical gases from being able to leak through such bores. In turn, in the case of the embodiment contemplating the combination of two devices 5 and 6, as may again be seen in the representation of Figure 6, as well in the representations of Figures 4 and 5, the upper device 5 is in all cases provided to include the same features as the formerly described device, whereas the lower device 6 is further provided with a horizontal inner conduit 22, whose inflow port 23 is plugged in a gas-tight manner by means of a plug-in cap 24, or the like, and is further fluidly communicating - via a vertical inner conduit 25 - with the inflow port 9 of the upper inner conduit 11, upstream to the valve member 12 with reference to the flow direction A of the medical gas, while the outflow port 26 of such horizontal inner conduit 22 is in turn plugged in a gas-tight manner by means of a plug-in cap 27, or the like, and is further fluidly communicating - via a further vertical inner conduit 28 - with the outflow port 10 of the upper inner conduit 11, downstream from the valve member 13 with reference to the flow direction A of the medical gas. Even in this embodiment of the inventive gas-pressure reducing and stabilizing device, the vertical inner conduits 25 and 28 are in fluid communication with the outside via related lower access bores 29 and 30 extending through the bottom surface 31 of the case-like body 8, which can be plugged in a gas-tight manner by means of corresponding plug-in caps 32 and 33, or the like, or can in turn be connected with an external emergency supply source of medical gas (not shown) and/or a highest and lowest allowable pressure detecting device (not shown, either), to the same purposes and for the same reasons as described afore in connection with the device 5.

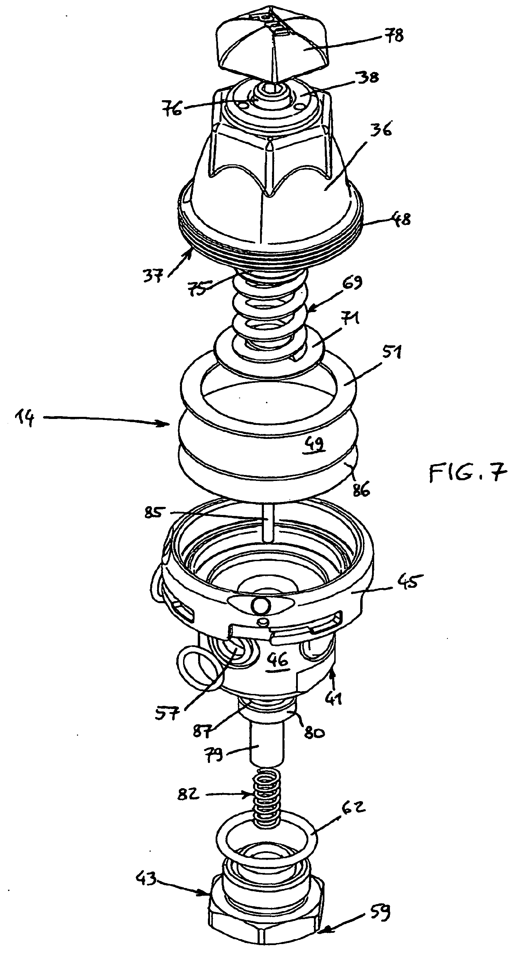

Finally, in both above-described embodiments of the inventive device, on the upper portion of the same device there are mounted two pressure gauges 34 and 35, which fluidly communicate on their lower side with the related inflow and outflow ports 9 and 10 to detect the level of the gas supply pressure that is due to be reduced, and the level of the gas distribution pressure that is reduced - in the manner that shall be described further on - as the gas passes through the gas-pressure regulating part 14 of the device, respectively. Referring now to Figures 7 to 9, which illustrate the gas-pressure regulating part 14 with the various component parts thereof, it can be noticed that such gas-pressure regulating part is substantially comprised of a circularly-shaped bell 36 gradually tapering off from the circular planar base 37 thereof to thereby form a front planar terminal wall 38 delimiting a corresponding inner cavity 39 that is open in said planar base 37 and is provided with a central through-bore 40 in the front planar terminal wall 38 thereof

The gas-pressure regulating part 14 is further comprised of a short cylindrical sleeve 41, which delimitates an inner through-cavity 42, and a base plug 43 provided with a central inner accommodation 44 extending down along part of the thickness thereof, wherein such sleeve is adapted to removably couple with the circularly-shaped bell 36 and the base plug is in turn adapted to removably couple with the above-cited cylindrical sleeve 41. The coupling of the cylindrical sleeve 41 with the circularly-shaped bell 36 is made possible by forming the connecting portion of said sleeve to include a circular widening-out rim 45 having a larger diameter than the remaining body 46 of the same sleeve, and by providing such circular widening-out rim with an internal threading 47, as well as by providing a further outer threading 48 extending along the outer surface of the circular base 37 of the bell 36, in a position corresponding to the position of the above-cited inner threading 47, so that such two component parts are able to be joined to each other by screwing said two threadings 47 and 48 with each other, under interposition of appropriate gaskets or other sealing material (not shown) to ensure gas tightness and, therefore, positively oppose the occurrence of any undesired gas leakage, and the respective inner cavities 39 and 42 of the bell 36 and the sleeve 41 are in this way fluidly communicating with each other. Anyway, these two component parts may of course be coupled together also in a different manner, such as for instance by having them snap-fitted or press-fitted together, or by any other appropriate joining system, without departing from the scope of the present invention. In this circumstance, in the inner cavities 39 and 42 there is placed at least one thin elastic membrane, e.g. of rubber or any other suitable elastomeric material, which extends to cover almost the whole width of the same inner cavities, such membrane being laid to rest upon a planar inner step-like configuration 50 of the cylindrical sleeve 41, which joins into the widening-out rim 45 of the same sleeve, under interposition of at least a ring-shaped sealing gasket 51, so that such membrane is ultimately effective in subdividing the upper inner cavity 39 from the lower inner cavity 42 in a gas-tight manner. In turn, the cylindrical sleeve 41 is provided with a circular inner rim 52, which joins into the above-cited circular widening-out rim 45 and terminates in an inner circular lip 53, directed radially inwards into the cavity 42 of the same sleeve, thereby narrowing the cross-section area thereof in this zone, wherein such inner circular rim 52 is provided with a ring-shaped slit forming the accommodation intended for receiving an end portion of a circular filter 55 of a kind as generally known as such in the art, which is provided there as a purifier for the medical gas being circulated, whereas the other end portion of such filter is accommodated in a corresponding slit or notch 56 provided in the base plug 43.

The cylindrical sleeve 41 is further provided with two through-bores 57 and 58 extending through the sleeve body 46 and angularly offset by an angle of 180° relative to each other, both of them fluidly communicating with the inner cavity 42 of the same sleeve, wherein such bores are sited upstream to the pressure-reducing zone of the pressure-regulating part 14, in which the medical gas flows into the inner cavity 42 at the standard supply pressure thereof, which has therefore to be reduced, and downstream from such pressure-reducing zone, respectively, in which the medical gas eventually flows out at a pressure that has been reduced at the desired level. The purpose of such bores is to either fluidly connect the inner cavity 42 to the related inner horizontal conduit 11 and/or 22 of the device, or - as the case may be - to cut off such fluid connection, as this shall be described further on, so as to enable or cut off the flow of medical gas through the same device towards the apparatuses provided to deliver the same gas to the patients under treatment. Finally, the base plug 43 is provided with a circular planar base 59 having a slightly smaller diameter than the sleeve body 46, and has a configuration tapering through two steps 60 and 61 lying over each other with a decreasing diameter, and the height of the plug 43 is such that, when it is fitted on - in the way that shall be explained further on - to a portion of the inner cavity 42 of the cylindrical sleeve body 46, the same plug is able to slip into the same inner cavity by only a limited extent.

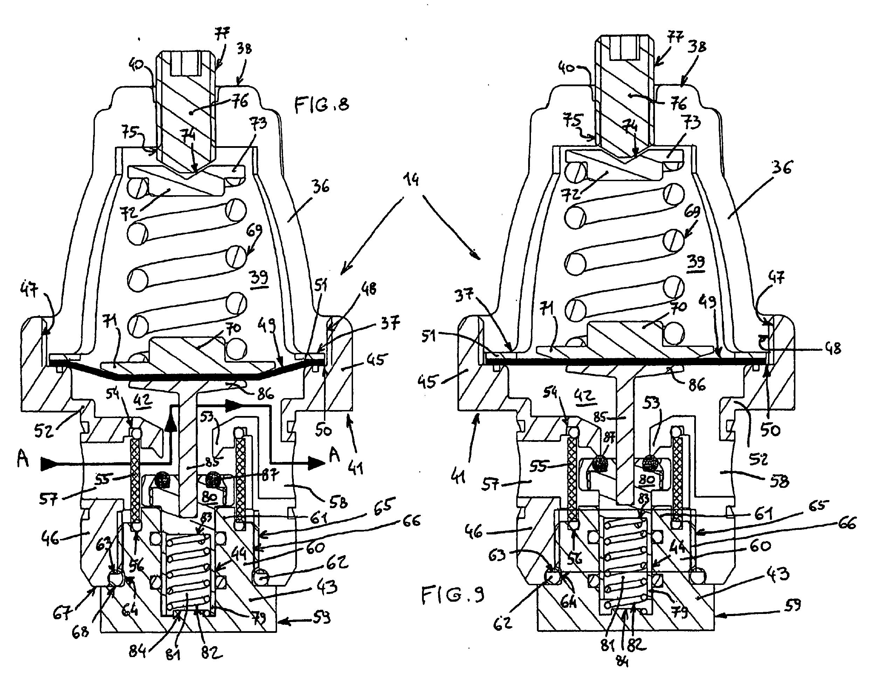

The coupling of the base plug 43 with the cylindrical sleeve body 46 is done by first of all fitting the base 59 of the plug in a corresponding inner receptacle (not shown) provided as an accommodation in the device according to the present invention, and placing a ring, bush or the like 62 of suitable anti-friction material, or even a bearing, into an appropriate accommodation provided between such base 59 of the plug and the sleeve 41 in the form of a circular groove 63 cut around the inlet opening of the inner cavity 42 of the sleeve 41 and a corresponding circular groove 64 cut around the base portion 59 of the plug. Then, the assembly formed of the bell 36 and the sleeve 41 is coupled on to the base plug 43 by pressing such assembly in an axial direction towards the base plug and rotating the same assembly - so as to bring about a gas-tight coupling, in the form of a bayonet-like, dovetail-like or similar joint, between the inner circular rim 65 of the sleeve body 46 and the outer circular rim 66 of the step 60 of the base plug 43 - until the outer rim 67 of the sleeve body 46 comes eventually into abutment against the opposite inner rim 68 of the base portion 59 of the plug, in a condition in which between such two component parts there is defined the afore-described accommodation adapted to receive said ring, bush or bearing, and in which the end portions of the circular filter 55 are accommodated in the circular groove 54 and the circular groove 56 provided in the step 61 of the base plug 43. In this manner, when these component parts are coupled to each other in the above-described manner, said assembly formed of the bell and the cylindrical sleeve is able to rotate by a pre-determined angle of rotation relative to the fixed base plug from a first operating position (to be seen in the lower portion of Figure 6) to a second operating position (to be seen in the upper portion of Figure 6), and vice-versa, wherein in the above-cited first operating position the inner cavity 42 of the sleeve 41 is fluidly connected - via the inflow through-bore 57 - to a portion of inner horizontal conduit 11 and/or 22, downstream from the first valve member 12, and - via the outflow through-bore 58 - to the remaining portion of inner horizontal conduit 11 and/or 22, upstream to the second valve member 13, and in this condition, when both said valve members 12 and 13 are opened, the medical gas can therefore flow through the inner conduit 11 and/or 22 and the inner cavity 42 to be delivered at a reduced pressure, whereas in the above-mentioned second operating position said through-bores 57 and 58 are rotated by 90° relative to the previous position, so that they are no longer fluidly communicating with the above-mentioned portions of conduit 11 and/or 22, in a condition in which - upon closure of the valve members 12 and 13 - the flow of medical gas through the device under consideration is shut off, thereby most reliably and securely preventing the medical gas from being able to flow further on towards the apparatuses provided to deliver it to the patients under treatment, so that the bell-and-sleeve assembly can be removed from the fixed base plug by performing the reverse sequence of the operations done to fit the same assembly in position, to thereby enabling any possible repair, maintenance and/or replacement of any component part of the device under consideration to be conveniently carried out as required. Regardless of the operating position in which such assembly may also be rotated, the same assembly is then secured - with the aid of some kind of dowel-like or similar contrivance - to the support structure 7 of the device being considered, so that it cannot displace from or move off such position unless specific manual operations are carried out. Referring now to Figures 8 and 9, it can be noticed that in the inner cavity 39 of the bell 36 there is housed at least one spring 69, whose end portion situated in the inlet opening side of the same inner cavity is secured around a protruding step 70 of a rigid disk 71, having a smaller diameter than the afore-cited inner cavity, which is in turn secured against the opposite surface of the elastic membrane 49, whereas the other end portion of such spring is secured around a protruding step 72 of a further rigid disk 73, situated close to the central through-bore 40 of the terminal wall 38 of said bell, and provided with central notch 74 in the surface lying opposite to the one in which the spring 69 is secured, said notch being adapted to receive and accommodate the corresponding terminal portion 75 of a screw 76, which is capable of being screwed in and out through the related inner threading of said through-bore 40, and whose head 77 is protruding above the terminal wall 38 of said bell and is covered by a cap 78. In turn, in the accommodation 44 provided in the base plug 43 there is received a corresponding cylindrical body 79, which features a circular head 80 facing the inner ring-shaped lip 53 of the cylindrical sleeve 41, and which is adapted to slide axially through a short length into said accommodation 44. The cylindrical body 79 is provided with a central blind hole 81, in which there is received a related spring 82, whose ends are abutting against the bottom wall 83 of the blind hole 81 of the cylindrical body 79 and the bottom wall 84 of the receptacle 44 of the base plug 43, respectively. In turn, the circular head 80 is situated within the circular filter 55, as slightly separated therefrom, and is further provided with a receptacle in which there is inserted and secured the elongated shaft 85 that is firmly joined to a rigid disc 86 being attached against the elastic membrane 49, such head being further so shaped as to be able to accommodate a sealing gasket 87 adapted to cooperate with the inner ring-shaped lip 53 of the cylindrical sleeve.

In this way, the regulation of the pressure of the medical gas flowing into the inner cavity 42 of the cylindrical sleeve 41, in the direction indicated by the arrow A, upon opening of the valve member 12 and following passage of the gas through the inflow bore 57 of the same sleeve and the circular filter, is performed by screwing the screw 76 in or out, as the case may be, and therefore varying the opposing force of the spring 69, which antagonizes the force of the pressure of the gas flowing into and circulating inside the above-mentioned inner cavity 42, which results in the elastic membrane 49 being instantly shifted in a continuous manner due to its being urged by the antagonistic forces of the spring 69 and the pressure of the inflowing gas that act against the same membrane, and this to the purpose of keeping the balance condition between such two antagonistic forces. In this condition, the above-described displacement of the elastic membrane 49 causes also the cylindrical body 79, and therefore also the head 80 of such cylindrical body, to correspondingly slide in a reciprocating mode in the respective accommodation 44 through a movement that is defined by the length existing from a fully opened operating position (see Figure 8), in which the head 80 is lowered to rest against the base plug 43, thereby compressing the spring 82, while the related sealing gasket 87 is shifted apart from the opposite inner ring-shaped lip 53 of the cylindrical sleeve 41, to a fully closed operating position (see Figure 9), in which the head 80 is raised and shifted apart from the base plug 43, thereby relieving the spring 82, while the related sealing gasket 87 is compressed against the above-cited lip 53, in the condition in which in the first case the medical gas flows into said inner cavity 42 and the pressure thereof is reduced according to the antagonistic force of the spring 69, and such flow of the gas towards to outflow port 58 takes place as long as the inflow port of the gas into said inner cavity 42 is open, albeit to a varying extent, and, in the second case, the medical gas does on the contrary not flow into the inner cavity 42, and therefore towards the outflow bore 58, any longer.

This continuous reciprocating sliding motion of the head 80 to and from the above-noted two operating positions is effective in continuously - i.e. instant by instant - not only the required reduction in the gas pressure, but also a stabilization of the pressure of the gas flowing at a reduced pressure from the pressure-regulating component part 14 of the device. The resulting inventive device is therefore able to perform the twofold function of both regulating/reducing/stabilizing the pressure of the gas being delivered to the patient and physically shutting out, i.e. stopping the flow of the gas, thanks to the rotation of the bell-sleeve assembly into the shut-off position to cut out the flow of the medical gas, in this case practically enabling the various valve units currently used in the art, as provided at the inflow ports of each gas distribution and delivery installation, to be simply done away with.

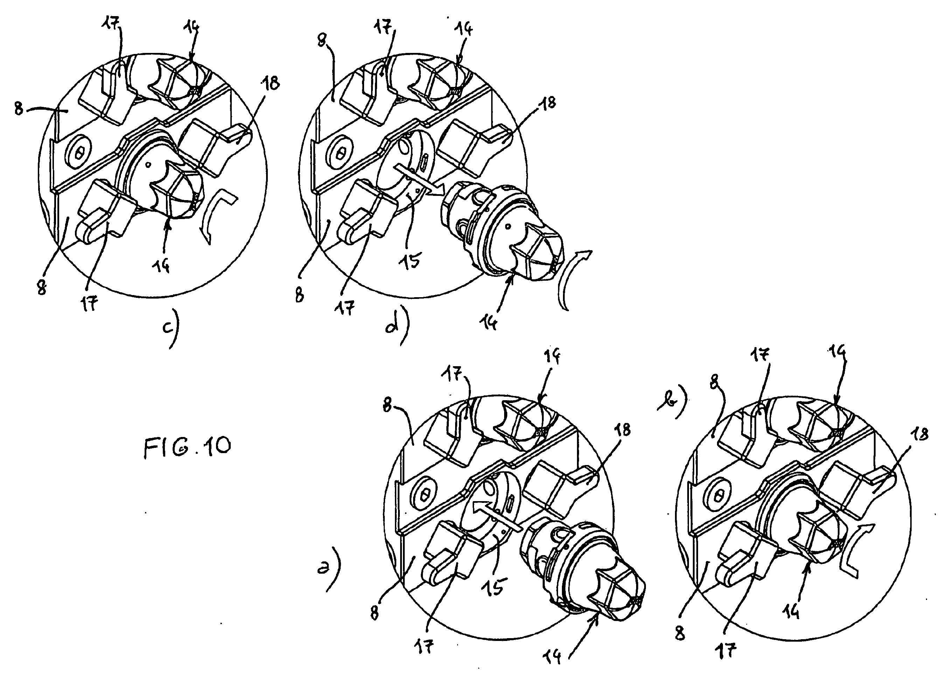

Finally, schematically illustrated in Figure 10 there are the various steps needed to fit and remove the pressure-regulating component part 14 into and from the case-like body 8 of each pressure reducing and stabilizing device according to the present invention.

## Claims

1. Gas-pressure reducing and stabilizing device (5 ; 5, 6) for use in connection with equipment designed for delivering gases of various kind for medical applications, adapted to be removably mounted in such gas delivery equipment for medical uses installed in health-care structures of surgery and medical treatment facilities, hospitals, clinics and the like, to the purpose of enabling such gases to be delivered at an appropriately reduced pressure to the patients under treatment through apparatuses of a traditional type, the device comprising a case-like body (8) housing first and second valve means (12, 13) connected to a conduit (9) of the gas supply installation supplying the medical gas at a high pressure and an outflow conduit (10) letting off the gas at a reduced pressure, respectively, the latter conduit being in turn connected with the respective apparatuses for the delivery of the gas to the patients, said valve means (12, 13) being adapted to enable the gas to flow or positively prevent the gas from flowing through the device (5 ; 5, 6), said case-like body (8) also comprising a removable gas-pressure regulation means (14) for reducing and regulating the pressure of the circulating gas and filter means (55) for purifying the medical gas being circulated therethrough, and further including gas-pressure measuring means (34, 35) at both the gas inflow and the gas outflow sides of the device, **characterized in that** said gas-pressure regulation means (14) comprise accommodation means (36, 41) comprising a circularly shaped bell (36) and a cylindrical sleeve (41), which are coupled to each other, and housed in a circularly-shaped accommodation (15) in a central position along an inner conduit (11) communicating with said gas supply conduit (9) and said outflow conduit (10) and provided with said first and second valve means (12, 13), located downstream from said gas supply conduit (9) and upstream to said outflow port (10), respectively, relative to the flow direction A of the medical gas circulating therethrough, wherein into said circularly shaped bell (36) and said cylindrical sleeve (41) there are mounted partition means (49) comprising at least one thin elastic membrane (49) of rubber or other suitable elastic material, adapted to subdivide in a gas-Light manner the interior of said gas-pressure regulation means into a first inner cavity (39) and a second inner cavity (42), wherein the latter fluidly communicates with said gas supply conduit (9) and said outflow conduit (10), said elastic membrane (49) being extended to cover almost the whole width of said first and second cavities (39, 42) and placed between said bell (36) and said cylindrical sleeve (41), under interposition of gas-tightly sealing means (ring-shaped sealing gasket 51), said first cavity (39) being provided with spring means (spring 69) acting upon said elastic membrane (49) with variable forces in opposition to the pressure of gas flowing into said second cavity (42), and said second cavity (42) being provided with said filter means (55) as well as with support means (43) capable of being removably coupled to both said case-like body (8) in a fixed position and said accommodating means (bell 36 and cylindrical sleeve 41), so as to enable the latter to be rotated by a given angle of rotation relative to said support means (43), said support means (43) being provided internally with shut-off means (79, 80) adapted to shut off the flow of the gas through said second inner cavity (42), sais shut-off means (79, 80) being associated with further spring means (82) and joined to said elastic membrane (49), so that the displacement of the latter, as caused by the pressure opposing forces exerted thereupon by said spring means (69) and the pressure of the gas flowing into and circulating through said second cavity (42), causes said gas-flow shut-off means (79, 80) to accordingly shift from an opening position allowing the gas to flow through said second inner cavity (42), and to be distributed at a reduced pressure to the related apparatuses for the delivery of the gas to the patients, to a closing position stopping the flow of gas therethrough and to said apparatuses ; the device being also **characterized in that** said supply conduit (9) and said outflow conduit (10) are also communicating with a vertical inner conduit (25, 28), which can be connected with an external emergency supply source of medical gas and/or a highest and lowest allowable pressure detecting device.

2. Device according to claim 1, **characterized in that** said circularly shaped bell (36) is tapered off gradually from the circular planar base (37) thereof to thereby form a front planar terminal wall (38), delimiting said first inner cavity (39) that is open in said planar base (37), and is provided with a central through-bore (40) in said front planar terminal wall (38) thereof, and said cylindrical sleeve (41) delimiting said second inner cavity (42) fluidly communicating with said gas supply and outflow conduits (9, 10) via at least one respective through-bore (57, 58), said through-bores (57, 58) being provided to extend through the sleeve body (46) as angularly offset by an angle of 180° relative to each other, and said cylindrical sleeve (41) being further provided with a ring-shaped inner rim (52), which joins into an inner circular lip (53) directed radially inwards into said second cavity (42), thereby narrowing the cross-section area thereof in this zone, said inner ring-shaped rim (52) being accommodating an end portion of said filter means (55), whereas the other end portion thereof is accommodated within said support means (43).

3. Device according to claim 2, **characterized in that** said spring means (69) comprise at least one spring (69), which is secured between said elastic membrane (49) and said front planar wall (38), and with which there cooperates at least one adjustment screw (76) capable of being screwed in and out through said central through-bore (40), to correspondingly vary the force exerted by said spring (69) against said elastic membrane (49).

4. Device according to claim 2, **characterized in that** said filter means comprise at least a circular filter (55) of a traditional kind.

5. Device according to claim 2, **characterized in that** said support means comprise a base plug (43) provided with a central inner accommodation (44), which extends down along part of the thickness thereof, and a circular planar base (59) having a slightly smaller diameter than said sleeve body (46) and a configuration tapering through two steps (60, 61) lying over each other with a decreasing diameter, said base plug (43) being coupled with said cylindrical sleeve (41) by fitting said steps (60, 61) into the base opening of said second inner cavity (42) and bringing said circular planar base (59) into abutment against the outer rim (67) of said sleeve body (46), under interposition of anti-friction means (62) between the same base plug (43) and said sleeve body (46).

6. Device according to claim 5, **characterized in that** said shut-off means comprise a cylindrical body (79) adapted to slide axially through a short length into said inner accommodation (44) and provided with an enlarged circular head (80), which faces said ring-shaped lip (53) and is provided with sealing means (gasket 87) adapted to cooperate with said ring-shaped lip (53) so as to be compressed thereagainst or relieved therefrom according to the position into which said cylindrical body (79) is shifted, following the corresponding displacement of said elastic membrane (49), whereupon the gas is therefore capable of or is prevented from flowing through said second inner cavity (42), said cylindrical body (79) being provided with a central blind hole (81), in which there are received said further spring means (spring 82), whose ends are abutting against the bottom wall (83) of said blind hole (81) and the bottom wall (84) of said accommodation (44) in said base plug (43), respectively.

## Patentansprüche

1. Gasdruckminder- und Stabilisierungseinrichtung (5;5,6) zur Verwendung in Verbindung mit einer Apparatur, die dazu bestimmt ist, Gase verschiedener Art für medizinische Zwecke zuzuführen, und geeignet ist, entfernbar in eine solche Gaszuführapparatur für medizinische Zwecke eingebaut zu sein, die in Gesundheitsstrukturen von Arztpraxen und medizinischen Behandlungseinrichtungen, Hospitalen, Kliniken und dergleichen installiert ist, zu dem Zweck, dass solche Gase mit einem geeignet reduzierten Druck den Patienten zugeführt werden können, die unter Behandlung sind, durch Geräte eines herkömmlichen Typs, wobei die Einrichtung einen Gehäuse-artigen Körper (8) enthält, in dem sich erste und zweite Ventilmittel (12,13) befinden, die mit einer Leitung (9) der Gaszufuhrinstallation verbunden sind, die das medizinische Gas mit einem hohen Druck liefert, sowie mit einer Ausflussleitung (10), die das Gas mit einem reduzierten Druck auslässt, wobei die letztere Leitung ihrerseits mit dem jeweiligen Gerät für die Zufuhr des Gases zu dem Patienten verbunden ist, wobei die Ventilmittel (12, 13) geeignet sind, das Gas strömen zu lassen oder das Gas zuverlässig am Strömen durch die Einrichtung (5;5,6) zu hindern, wobei der Gehäuse-artige Körper (8) auch ein entfernbares Gas-Druck-Regulierungsmittel (14) aufweist, um den Druck des zirkulierenden Gases zu reduzieren und zu regulieren, und Filtermittel (55), um das medizinische Gas zu reinigen, das hindurch zirkuliert, und ferner Gas-Druck-Messmittel (34,35) sowohl an der Gaseintrittsseite als auch der Gasauströmseite der Einrichtung aufweist, **dadurch gekennzeichnet, dass** die Gas-Druck-Regulierungsmittel (14) Aufnahmemittel (36,41) enthalten, die eine kreisrund geformte Glocke (36) und eine zylindrische Hülse (41) aufweisen, die miteinander gekoppelt sind, und in einer kreisrund geformten Aufnahme (15) in einer mittigen Position entlang einer inneren Leitung (11) untergebracht sind, die mit der Gaszuführleitung (9) und der Gasausströmleitung (10) verbunden ist, und mit dem ersten und den zweiten Ventilmittel (12,13) stromabwärts der Gaszuführleitung (9) und stromaufwärts der Gasausströmleitung (10) versehen ist, relativ zu der Strömungsrichtung A des hindurch zirkulierenden medizinischen Gases, wobei in die kreisrund geformte Glocke (36) und die zylindrische Hülse (41) Trennmittel (49) eingebaut sind, die wenigstens eine dünne elastische Membran (49) aus Gummi oder einem anderen geeigneten elastischen Material enthalten, und geeignet sind, auf eine gasdichte Weise das Innere der Gas-Druck-Regulierungsmittel in einen ersten inneren Hohlraum (39) und einen zweiten inneren Hohlraum (42) zu unterteilen, wobei der letztere in Strömungsverbindung mit der Gaszuführleitung (9) und der Gasausströmleitung (10) steht, wobei sich die elastische Membran (49) so erstreckt, dass sie beinahe die gesamte Breite des ersten und des zweiten Hohlraums (39,42) überdeckt und zwischen der Glocke (36) und der zylindrischen Hülse (41) angeordnet ist, unter Zwischenschaltung eines gasdichten Dichtungsmittels (ringförmige Dichtungsscheibe (51)), wobei der erste Hohlraum (39) mit einem Federmittel versehen ist (Feder 69), das mit veränderbaren Kräften entgegen dem Druck des Gases, das in den zweiten Hohlraum (42) fließt, auf die elastische Membran (49) einwirkt, und wobei der zweite Hohlraum (42) mit dem Filtermittel (55) sowie mit Haltemitteln (43) versehen ist, die geeignet sind, sowohl mit dem Gehäuse-artigen Körper (8) in einer festen Position und den Aufnahmemitteln (Glocke 36 und zylindrische Hülse (41)) entfernbar gekoppelt zu werden, so dass die letzteren in der Lage sind, um einen vorgegebenen Drehwinkel gegenüber den Haltemitteln (43) gedreht zu werden, wobei die Haltemittel (43) innen mit Absperrmitteln (79, 80) versehen sind, die den Strom des Gases durch den zweiten inneren Hohlraum (42) absperren können, wobei die Absperrmittel (79, 80) mit einem weiteren Federmittel (82) versehen und mit der elastischen Membran (49) verbunden sind, so dass die Verlagerung der letzteren, verursacht durch dem Druck entgegengesetzte Kräfte, die von dem Federmittel (69) und dem Druck des Gases ausgeübt werden, das in dem zweiten Hohlraum (42) fließt und zirkuliert, bewirkt, dass die Gas-Strom-Absperrmittel (79, 80) entsprechend von einer Öffnungsposition, die den Gasstrom durch den zweiten inneren Hohlraum (42) zulässt, um mit einem verringerten Druck zu der zugehörigen Apparatur zur Zufuhr von Gas zu den Patienten verteilt zu werden, in eine Schließposition verlagert werden, die den Gasstrom hindurch und zu der Apparatur stoppt, wobei die Einrichtung außerdem **dadurch gekennzeichnet ist, dass** die Zuführleitung (9) und die Ausströmleitung (10) außerdem mit einer vertikalen inneren Leitung (25, 28) in Verbindung stehen, die mit einer äußeren Notfallzuführquelle von medizinischem Gas und/oder einer Erfassungseinrichtung für höchsten und niedrigsten zulässigen Druck verbunden sein kann.

2. Einrichtung nach Anspruch 1, **dadurch gekennzeichnet, dass** die kreisrund geformte Glocke (36) zunehmend von einer kreisrunden ebenen Basis (37) abgeschrägt ist, um eine vordere ebene Endwand (38) zu bilden, um den ersten inneren Hohlraum (39) zu begrenzen, der in der ebenen Basis (37) offen ist und mit einer zentralen Durchgangsbohrung (40) in der vorderen ebenen Endwand (38) versehen ist, und dass die zylindrische Hülse (41) den zweiten inneren Hohlraum (42) begrenzt, der in Fluidverbindung mit der Gaszuführleitung und der Gasausströmleitung (9,10) über wenigstens eine zugehörige Durchgangsbohrung (57, 58) steht, wobei die Durchgangsbohrungen (57, 58) sich durch den Hülsenkörper (46) so erstrecken, dass sie um einen Winkel von 180° relativ zueinander winkelförmig versetzt sind, und wobei die zylindrische Hülse (41) außerdem mit einem ringförmigen inneren Rand 52 versehen ist, der in eine innere kreisförmige Lippe (53) übergeht, direkt radial einwärts in den zweiten Hohlraum (42), wodurch die Querschnittsfläche in dieser Zone verengt wird, wobei der innere ringförmige Rand (52) einen Endabschnitt des Filtermittels (55) aufnimmt, während der andere Endabschnitt davon in das Haltemittel (43) aufgenommen ist.

3. Einrichtung nach Anspruch 2, **dadurch gekennzeichnet, dass** Federmittel wenigstens eine Feder (69) enthalten, die zwischen der elastischen Membran (49) und der vorderen ebenen Wand (38) befestigt ist und mit der wenigstens eine Einstellschraube (76) zusammenwirkt, die durch die zentrale Durchgangsbohrung (40) ein und aus geschraubt werden kann, um entsprechend die Kraft zu variieren, die von der Feder (69) gegen die elastische Membran (49) ausgeübt wird.

4. Einrichtung nach Anspruch 2, **dadurch gekennzeichnet, dass** das Filtermittel wenigstens einen kreisförmigen Filter (55) einer herkömmlichen Art enthält.

5. Einrichtung nach Anspruch 2, **dadurch gekennzeichnet, dass** das Haltemittel einen Basisstopfen (43) mit einer mittigen inneren Aufnahme (44) aufweist, die sich entlang eines Teils seiner Dicke nach unten erstreckt, und dass eine kreisförmige ebene Basis (59) einen etwas kleineren Durchmesser hat als der Hülsenkörper (46) und eine Konfiguration, die durch zwei Stufen (60, 61) verringert ist, die übereinander mit einem abnehmenden Durchmesser liegen, wobei der Basisstopfen (43) mit der zylindrischen Hülse (41) dadurch gekoppelt ist, dass diese Stufen (60,61) in die Basisöffnung des zweiten inneren Hohlraums (42) passen und die kreisrunde ebene Basis (59) in Anlage an den äußeren Rand (47) des Hülsenkörpers (46) bringen, unter Zwischenschaltung von Anti-Reibungsmitteln (62) zwischen dem Basisstopfen (43) und dem Hülsenkörper (46).

6. Einrichtung nach Anspruch 5, **dadurch gekennzeichnet, dass** die Absperrmittel einen zylindrischen Körper (79) aufweisen, der axial über eine kurze Länge in die innere Aufnahme (44) gleiten kann und mit einem vergrößerten kreisrunden Kopf (80) versehen ist, der der ringförmigen Lippe (53) gegenüber liegt und mit Dichtungsmitteln (Scheibe 87) versehen ist, die mit der ringförmigen Lippe (53) zusammenwirken kann, indem sie dagegen gepresst oder davon entfernt wird entsprechend der Position, in die der zylindrische Körper (79) bewegt wird, gefolgt von der entsprechenden Verlagerung der elastischen Membran (49), woraufhin das Gas durch den zweiten inneren Hohlraum (42) fließen kann oder daran gehindert ist, wobei der zylindrische Körper (79) mit einer mittigen Blindbohrung (81) versehen ist, in die weitere Federmittel (Feder 82) aufgenommen sind, deren Enden an der Bodenwand (83) der Blindbohrung (81) und der Bodenwand (84) der Aufnahme (44) in dem Basisstopfen (43) anliegen.

## Revendications

1. Dispositif de réduction et de stabilisation d'une pression gazeuse (5 ; 5, 6) destiné à être utilisé en relation avec un équipement conçu pour fournir des gaz de différents types pour des applications médicales, adapté pour être monté de manière amovible dans un tel équipement d'alimentation en gaz à usage médical installé dans des structures de soins d'établissements de chirurgie et de traitement médical, d'hôpitaux, de cabinets médicaux, etc., afin de permettre à de tels gaz d'être fournis à une pression correctement réduite aux patients sous traitement par le biais d'appareils classiques, le dispositif comprenant un corps en forme de boîtier (8) qui contient des premier et second moyens de soupapes (12, 13) reliés à une conduite (9) de l'installation d'alimentation en gaz fournissant le gaz médical à une pression élevée et à une conduite d'évacuation (10) évacuant le gaz à une pression réduite, respectivement, cette dernière conduite étant à son tour reliée aux appareils respectifs destinés à fournir le gaz aux patients, lesdits moyens de soupapes (12, 13) étant adaptés pour permettre au gaz de circuler ou pour empêcher positivement le gaz de circuler dans le dispositif (5 ; 5, 6), ledit corps en forme de boîtier (8) comprenant également un moyen de régulation de la pression gazeuse amovible (14) destiné à réduire et à réguler la pression du gaz circulant, et un moyen de filtrage (55) destiné à purifier le gaz médical en circulation à l'intérieur, et comprenant en outre un moyen de mesure de la pression gazeuse (34, 35) au niveau de l'admission et de l'évacuation de gaz du dispositif, **caractérisé en ce que** ledit moyen de régulation de la pression gazeuse (14) comprend un moyen de logement (36, 41) qui comprend une cloche de forme circulaire (36) et un manchon cylindrique (41), qui sont reliés l'un à l'autre, et contenus dans un logement de forme circulaire (15) à un emplacement central le long d'une conduite intérieure (11) communiquant avec ladite conduite d'alimentation en gaz (9) et ladite conduite d'évacuation (10), et munis desdits premier et second moyens de soupapes (12, 13), en aval de ladite conduite d'alimentation en gaz (9) et en amont dudit orifice d'évacuation (10), respectivement, par rapport au sens d'écoulement A du gaz médical qui circule à l'intérieur, dans lequel, dans ladite cloche de forme circulaire (36) et ledit manchon cylindrique (41), sont placés des moyens de séparation (49) qui comprennent au moins une fine membrane élastique (49) en caoutchouc ou tout autre matériau élastique approprié, adaptés pour subdiviser de manière étanche au gaz l'intérieur dudit moyen de régulation de la pression gazeuse en une première cavité intérieure (39) et une seconde cavité intérieure (42), dans lequel cette dernière est en communication de fluide avec ladite conduite d'alimentation en gaz (9) et ladite conduite d'évacuation (10), ladite membrane élastique (49) s'étendant afin de recouvrir la quasi-totalité de la largeur desdites première et seconde cavités (39, 42), et étant placée entre ladite cloche (36) et ledit manchon cylindrique (41), sous l'interposition d'un moyen d'étanchéité au gaz (joint d'étanchéité en forme de bague 51), ladite première cavité (39) étant munie d'un moyen formant ressort (ressort 69) qui agit sur ladite membrane élastique (49) avec des forces variables opposées à la pression du gaz qui circule dans ladite seconde cavité (42), et ladite seconde cavité (42) étant munie dudit moyen de filtrage (55) et d'un moyen de support (43) pouvant être relié de manière amovible audit corps en forme de boîtier (8) à un emplacement fixe, et audit moyen de logement (cloche 36 et manchon cylindrique 41), afin que ce dernier puisse être tourné selon un angle donné de rotation par rapport audit moyen de support (43), ledit moyen de support (43) étant muni à l'intérieur d'un moyen d'obturation (79, 80) adapté pour stopper l'écoulement de gaz à travers ladite seconde cavité intérieure (42), ledit moyen d'obturation (79, 80) étant associé à un autre moyen formant ressort (82) et joint à ladite membrane élastique (49), afin que le déplacement de cette dernière, provoqué par la pression opposée aux forces exercées dessus par ledit moyen formant ressort (69) et la pression du gaz circulant à travers ladite seconde cavité (42), permette audit moyen d'obturation d'écoulement de gaz (79, 80) de passer d'une position ouverte permettant au gaz de circuler à travers ladite seconde cavité intérieure (42), afin d'être distribué à une pression réduite aux appareils associés destinés à fournir le gaz aux patients, à une position fermée qui stoppe l'écoulement de gaz à l'intérieur et vers lesdits appareils ; le dispositif étant également **caractérisé en ce que** ladite conduite d'alimentation (9) et ladite conduite d'évacuation (10) communiquent également avec une conduite intérieure verticale (25, 28) qui peut être reliée à une source d'alimentation en gaz médical externe de secours et/ou à un dispositif de détection de pression maximale et minimale autorisée.

2. Dispositif selon la revendication 1, **caractérisé en ce que** ladite cloche de forme circulaire (36) est progressivement effilée depuis sa base plane circulaire (37) afin de former une paroi terminale plane avant (38), délimitant ladite première cavité intérieure (39) qui est ouverte dans ladite base plane (37), et est munie d'un trou traversant central (40) dans ladite paroi terminale plane avant (38), et ledit manchon cylindrique (41) délimitant ladite seconde cavité intérieure (42) est en communication de fluide avec lesdites conduites d'alimentation en gaz et d'évacuation (9, 10) via au moins un trou traversant respectif (57, 58), lesdits trous traversants (57, 58) étant prévus pour s'étendre à travers le corps du manchon (46) en étant décalés selon un angle de 180° l'un par rapport à l'autre, et ledit manchon cylindrique (41) étant en outre muni d'un bord intérieur en forme de bague (52), qui est joint à une lèvre circulaire intérieure (53) orientée radialement vers l'intérieur dans ladite seconde cavité (42), ce qui rétrécit sa surface transversale dans cette zone, ledit bord intérieur en forme de bague (52) contenant une partie d'extrémité dudit moyen de filtrage (55), tandis que l'autre partie d'extrémité de celui-ci est contenue dans ledit moyen de support (43).

3. Dispositif selon la revendication 2, **caractérisé en ce que** ledit moyen formant ressort (69) comprend au moins un ressort (69), fixé entre ladite membrane élastique (49) et ladite paroi plane avant (38), et avec lequel coopère au moins une vis de réglage (76) pouvant être vissée dans et dévissée dudit trou traversant central (40), afin de faire varier de manière correspondante la force exercée par ledit ressort (69) contre ladite membrane élastique (49).

4. Dispositif selon la revendication 2, **caractérisé en ce que** ledit moyen de filtrage comprend au moins un filtre circulaire (55) de type classique.

5. Dispositif selon la revendication 2, **caractérisé en ce que** ledit moyen de support comprend une fiche de base (43) munie d'un logement intérieur central (44), qui s'étend le long d'une partie de son épaisseur, et une base plane circulaire (59) ayant un diamètre légèrement inférieur audit corps de manchon (46) et une configuration qui s'effile sur deux niveaux (60, 61) se chevauchant avec un diamètre décroissant, ladite fiche de base (43) étant reliée audit manchon cylindrique (41) en plaçant lesdits niveaux (60, 61) dans l'ouverture de base de ladite seconde cavité intérieure (42) et en amenant ladite base plane circulaire (59) en butée contre le bord extérieur (67) dudit corps de manchon (46), sous l'interposition d'un moyen anti-frottement (62) entre la même fiche de base (43) et ledit corps de manchon (46).

6. Dispositif selon la revendication 5, **caractérisé en ce que** ledit moyen d'obturation comprend un corps cylindrique (79) adapté pour coulisser axialement sur une courte longueur dans ledit logement intérieur (44) et muni d'une tête circulaire élargie (80), qui fait face à ladite lèvre en forme de bague (53) et est munie d'un moyen d'étanchéité (joint 87) adapté pour coopérer avec ladite lèvre en forme de bague (53) afin d'être comprimé contre celle-ci ou détaché de celle-ci selon la position dans laquelle est placé ledit corps cylindrique (79), en suivant le déplacement correspondant de ladite membrane élastique (49), moyennant quoi le gaz peut ainsi circuler ou non à travers ladite seconde cavité intérieure (42), ledit corps cylindrique (79) étant muni d'un trou borgne central (81), dans lequel sont reçus ledit autre moyen formant ressort (82), dont les extrémités butent contre la paroi inférieure (83) dudit trou borgne (81), et la paroi inférieure (84) dudit logement (44) dans ladite fiche de base (43), respectivement.
